# EUROPÄISCHE PATENTSCHRIFT

(11) **EP 1 940 321 B1**
(45) Veröffentlichungstag und Bekanntmachung des Hinweises auf die Patenterteilung: **15.12.2010**
(21) Anmeldenummer: 06828826.5
(22) Anmeldetag: 17.10.2006
(51) Int. Cl.: A61F 2/24

(54) **VORRICHTUNG ZUR IMPLANTATION UND BEFESTIGUNG VON HERZKLAPPENPROTHESEN**
DEVICE FOR IMPLANTING AND FASTENING HEART VALVE PROSTHESES
DISPOSITIF POUR IMPLANTER ET FIXER DES VALVULES PROTHETIQUES

(30) Priorität: 28.10.2005 DE 102005051849
(43) Veröffentlichungstag der Anmeldung: 09.07.2008
(73) Patentinhaber: JenaValve Technology Inc., Wilmington, DE 19801 (US)
(72) Erfinder: FERRARI, Markus, 07747 Jena (DE); FIGULLA, Hans-Reiner, 07749 Jena (DE); DAMM, Christoph, 07743 Jena (DE)
(74) Vertreter: Trinks, Ole
(86) Internationale Anmeldenummer: PCT/EP2006/010023
(87) Internationale Veröffentlichungsnummer: WO 2007/048529

(56) Entgegenhaltungen:
- WO-A-2005/062980
- WO-A-2006/127756
- DE-A1- 10 301 026
- US-A1- 2002 151 970

## Beschreibung

Die vorliegende Erfindung betrifft eine Vorrichtung zur transvaskulären Implantation und Befestigung von Herzklappenprothesen, mit einem selbstexpandierenden Herzklappenstent, der an seinem proximalen Ende eine Herzklappenprothese aufweist.

Eine derartige Vorrichtung ist dem Grunde nach aus der Medizintechnik bekannt. Gegenwärtig stehen für den Ersatz menschlicher Herzklappen biologische oder mechanische Klappenmodelle zur Verfügung, die in der Regel über eine Öffnung des Brustkorbs chirurgisch nach Entfernung der kranken Herzklappe im Herzklappenbett festgenäht werden. Für diesen Eingriff muss der Kreislauf des Patienten durch eine Herz-Lungen-Maschine getragen werden, wobei während der Implantation der Herzklappenprothese ein Herzstillstand induziert wird. Dabei handelt es sich um einen risikoreichen chirurgischen Eingriff mit entsprechenden Risiken für den Patienten und mit einer langen postoperativen Behandlungsphase. Insbesondere kann ein solcher Eingriff bei herzschwachen Patienten nicht mehr durchgeführt werden.

In jüngster Zeit wurden minimal-invasive Therapieverfahren entwickelt, die sich insbesondere dadurch auszeichnen, dass für den Eingriff nur noch eine deutlich reduzierte Narkosezeit von Nöten ist. Ein Ansatz sieht dabei vor, eine selbstexpandierende Herzklappenprothese mit einer künstlichen Herzklappe und mit einem mit der Herzklappe verbundenen zusammenfaltbaren und expandierbaren Stent über ein entsprechendes Kathetersystem im menschlichen Körper zu implantieren. Eine solche selbstexpandierende Herzklappenprothese kann mit Hilfe eines Kathetersystems durch eine Leistenarterie oder -vene bis hin zum Implantationsort am Herzen geführt werden. Nach Erreichen des Implantationsortes kann dann der Stent, der in seiner Längsrichtung beispielsweise aus mehreren relativ zueinander abwinkelbaren selbstexpandierenden Stent-Segmenten zusammengesetzt ist, sukzessiv entfaltet werden. Nach der Entfaltung kann die Herzklappenprothese beispielsweise mit Unterstützung von Verankerungshaken zumindest im herznahen Bereich im jeweiligen Blutgefäß verankert werden. Die eigentliche Herzklappenprothese befindet sich dabei unmittelbar im proximalen Bereich des Stents.

Beispielsweise ist aus der Druckschrift DE 100 10 074 A1 eine Vorrichtung zur Befestigung und Verankerung von Herzklappenprothesen bekannt, die im wesentlichen aus drahtförmigen, miteinander verbundenen Elementen gebildet ist. Dabei ist vorgesehen, dass unterschiedliche Bügel eingesetzt werden, um eine sichere Befestigung und Abstützung der Herzklappenprothese zu erreichten. Die in dieser Druckschrift beschriebene Vorrichtung verwendet dazu drei jeweils gleiche Paare von Bügeln, die in jeweils einem Abstand von 120° zueinander angeordnet sind. Diese Bügel sind miteinander durch Festkörpergelenke verbunden, wobei die Festkörpergelenke die Funktion von Drehlagern erfüllen. Zusätzlich sind entgegengesetzt gebogene Bügel vorhanden, die möglichst gleichlange Hebelarme bilden, um eine sichere Anlage der Bügel auch bei peristaltischen Bewegungen am Herzen und Blutgefäß und eine sichere Abdichtung einer implantierten und fixierten Herzklappenprothese erreichen zu können.

Nächster Stand der Technik ist WO 2005/062980 offenbart eine Herzklappenprothese bestehend aus Positionsstent und Herzklappenstent, wobei der Herzklappenstent nicht explantierbar ausgebildet ist.

Druckschrift WO2006/127756, welches nach Art. 54(3) EPÜ für die Neuheit berücksichtigt werden muss, offenbart eine Herzklappenprothese, bestehend aus Positionsstent und Herzklappenstent, wobei der Herzklappenstent explantierbar ist.

Bei den bekannten Lösungen besteht jedoch die Gefahr einer Fehlimplantation von Herzklappen. Dies betrifft im wesentlichen die exakte Positionierung und angulare Ausrichtung von der zu implantierenden Herzklappenprothese. Insbesondere ist es - wenn überhaupt - nur mit großem Geschick des Behandlers möglich, einen Stent, der in seinem proximalen Ende die Herzklappenprothese aufweist, so genau in der Nähe der kranken Herzklappe des Patienten zu positionieren, dass sowohl eine hinreichende laterale Positionsgenauigkeit als auch eine geeignete angulare Lage der Herzklappenprothese optimal sichergestellt werden können. Auch eignen sich die bekannten Lösungen nur bedingt, nicht richtig bzw. fehlerhaft positionierte Herzklappenprothesen wieder zu explantieren; dies ist in der Regel nur noch mit hohem Aufwand möglich; insbesondere ist hierfür ein weiterer operativer Eingriff erforderlich.

Eine Fehlimplantation einer beispielsweise nicht optimal positionierten Herzklappenprothese kann unter anderem zu einer Undichtigkeit bzw. Klappeninsuffizienz führen, was erhebliche Belastungen des Ventrikels nach sich zieht. Erfolgt beispielsweise eine Implantation einer Herzklappenprothese zu weit oberhalb der eigentlichen Herzklappenebene, kann es zum Verschluss der Abgänge der Herzkranzgefäße (Koronarien) und damit zu einer tödlichen Koronarieschämie mit Herzinfarkt kommen. Demnach ist es zwingend erforderlich, dass sowohl die laterale Positionsgenauigkeit als auch die angulare Lage einer implantierten Herzklappenprothese den Erfordernissen entspricht.

Bei herkömmlichen Implantationstechniken, bei denen selbstexpandierbare Herzklappenprothesen durch beispielsweise eine Leistenarterie des Patienten minimalinvasiv bis an den Implantationsort am Herzen geführt werden, erfolgt die Einführung der Prothese üblicherweise mittels eines Führungsdrahtes und mit Hilfe von Kathetern, wobei auch herkömmliche Ballonkatheter eingesetzt werden können. Obwohl bei einem solchen Eingriff das Einführen, etwa mit Hilfe einer Röntgendurchleuchtung (Herzkatheterlabor = HKL) oder mit Hilfe von Ultraschall (transösophageales Echokardiogramm = TEE), überwacht und gesteuert werden kann, ist es - infolge der eingeschränkten Manövrierfähigkeit der beim Einführvorgang noch zusammengefalteten Herzklappenprothese, die trotz ihres zusammengefalteten Zustandes relativ große Dimensionen aufweist - häufig nicht möglich, die erforderlich Positionsgenauigkeit und insbesondere die angulare Lage der zu implantierenden Herzklappenprothese mit den entsprechend daran befestigten Befestigungselementen sicherzustellen. Insbesondere kann - infolge eines möglichen Verschlusses von Herzkranzgefäßen - ein Winkelversatz der implantierten Herzklappenprothese vom optimalen Implantationsort eine Gefährdung für den jeweiligen Patienten darstellen.

Bei der Auslegung einer Herzklappenprothese sind insbesondere auch die während der Füllungsphase des Herzzyklusses (Diastole) an der Prothese wirkenden, erhebliche Kräfte zu berücksichtigen, wonach eine sichere Verankerung erforderlich ist, um ein Ablösen der implantierten Herzklappenprothese zu verhindern.

Von daher muss sich einerseits die Herzklappenprothese bei dem Implantationsvorgang möglichst gut im entsprechenden Herzkranzgefäß manövrieren lassen, um somit eine optimale Positionsgenauigkeit sicherzustellen, und andererseits muss sich die implantierte Prothese in ihrem Implantationsort fest verankern lassen, um einen nachträglichen Versatz der Prothese wirksam verhindern zu können.

Der vorliegenden Erfindung liegt die Problemstellung zugrunde, dass sich bekannten Vorrichtung zur transvaskulären Implantation und Befestigung von Herzklappenprothesen häufig nicht eignen, auf einfache Weise eine Herzklappenprothese mit der notwendigen Positioniergenauigkeit an einer Herzkammer eines Patienten zu implantieren. Insbesondere können die erforderliche laterale Positionsgenauigkeit und angulare Lage der Herzklappenprothese in der Regel nur dann hinreichend sichergestellt werden, wenn der Behandler über eine entsprechende Erfahrung verfügt. Andererseits ist es bisher, wenn überhaupt, nur mit hohem Aufwand möglich, eine bereits implantierte Herzklappenprothese mit einem minimal-invasiven Eingriff zu explantieren oder eine fehlerhaft positionierte Herzklappenprothese entsprechend zu korrigieren.

Ausgehend von dieser Problemstellung liegt der vorliegenden Erfindung die Aufgabe zugrunde, eine Vorrichtung anzugeben, die es ermöglicht, auf möglichst einfache Weise eine Herzklappenprothese mit einem minimal-invasiven Eingriff in einem Patienten zu implantieren, wobei insbesondere eine erhöhte Positioniergenauigkeit der Prothese an der Herzkammer des Patienten sichergestellt werden kann. Insbesondere soll mit einer solchen Vorrichtung das Risiko einer fehlerhaften Implantation möglichst reduziert werden.

Erfindungsgemäß wird diese Aufgabe mit einer Vorrichtung nach Anspruch 1 gelöst.

Die erfindungsgemäße Vorrichtung weist eine ganze Reihe wesentlicher Vorteile gegenüber den aus dem Stand der Technik bekannten und vorstehend erläuterten Herzklappenprothesen auf. Durch die zweiteilige Ausbildung der Vorrichtung in der Gestalt des Herzklappenstents und des separat hiervon ausgebildeten Positionsstents kann insbesondere die Positioniergenauigkeit der Herzklappenprothese an der Herzkammer des Patienten deutlich erhöht werden. Der Positionsstent übernimmt hierbei in erster Linie die Funktion der Festlegung der Position der Herzklappenprothese an der Herzkammer des Patienten sowie die Funktion der Verankerung bzw. Befestigung der Prothese in ihrem optimalen Implantationsort. Insbesondere ist die Herzklappenprothese nicht an bzw. in dem Positionsstent sondern an dem separat von dem Positionsstent ausgeführten Herzklappenstent ausgebildet. Dies hat den Vorteil, dass die Dimension des Positionsstents im zusammengefalteten Zustand äußerst klein ist, was die Manövrierfähigkeit des Stents erhöht.

Der Herzklappenstent dient bei der erfindungsgemäßen Vorrichtung in erster Linie nur als Trägerstruktur für die zu implantierende Herzklappenprothese. Durch diese Funktionsteilung ist es möglich, sowohl den Positionsstent als auch den Herzklappenstent relativ einfach auszubilden. Insbesondere kann erreicht werden, dass im Vergleich zu einem Stent, an dem sowohl eine Herzklappenprothese als auch Mittel zum Positionieren und Befestigen der Herzklappenprothese angeordnet sind, der Positionsstent in seinem zusammengefalteten Zustand nur relativ geringe Dimensionen aufweist. Das Einsetzen des Positionsstents in der Arterie des Patienten ist somit - infolge der dadurch erreichten besseren Manövrierfähigkeit - wesentlich einfacher möglich. Eine erhöhte Positioniergenauigkeit des Positionsstents ist eine direkte Folge hiervon.

Die erfindungsgemäße Vorrichtung ist derart ausgeführt, dass erst nach dem Einbringen des Positionsstents in die Arterie des Patienten und nach dem Ausrichten des Stents bezüglich einer vorab festlegbaren axialen Rotation und horizontalen Position im Hinblick auf eine (alte) Herzklappe des Patienten der separat von dem Positionsstent ausgeführte Herzklappenstent in die Arterie oder Vene eingebracht wird. Der Herzklappenstent, welcher in seinem proximalen Ende die Herzklappenprothese aufweist, orientiert sich beim Einführvorgang selbständig an dem genau positionierten und an der Arterienwand fixierten Positionsstent. Im einzelnen wird der Herzklappenstent in dem expandierten Positionsstent selbständig in seine anhand des Positionsstents vorgegebene Implantationsposition geführt, in welcher die Herzklappenprothese im Hinblick auf die alte Herzklappe des Patienten optimal zu liegen kommt. Nachdem der Herzklappenstent seine mit Hilfe des Positionsstents vorgegebenen Position hinsichtlich der alten Herzklappe im Herzkranzgefäß eingenommen hat, wird durch beispielsweise eine externe Manipulation die vollständige Expansion des Herzklappenstents induziert, infolgedessen der Herzklappenstent erfindungsgemäß derart in Wechselwirkung mit dem Positionsstent tritt, dass der Herzklappenstent, und somit auch die in seinem proximalen Ende vorgesehene Herzklappenprothese, in der Implantationsposition positionell fixiert wird. Demnach dient der Positionsstent - neben der bereits erwähnten Funktion der Festlegung der Position der Herzklappenprothese an der Herzkammer des Patienten sowie die Funktion der Verankerung bzw. Befestigung der Prothese an dieser Position - auch die Funktion der Führung des Herzklappenstents beim Implantationsvorgangs in die für die Herzklappenprothese optimale Position. Die mit der erfindungsgemäßen Vorrichtung erzielbaren Vorteile liegen auf der Hand: insbesondere wird eine optimale Positionierung der Herzklappenprothese in ihrer endgültigen Implantationsposition ermöglicht, wobei das Ausrichten und Fixieren der Herzklappenprothese aufgrund des Zusammenwirkens des Herzklappenstents mit dem Positionsstent selbständig erfolgt. Einerseits kann hiermit eine positionsbedingte, fehlerhafte Implantation der Herzklappenprothese ausgeschlossen werden. Andererseits zeichnet sich die Vorrichtung dadurch aus, dass die Implantation und Befestigung der Herzklappenprothese auf besonders einfache Weise erfolgen kann.

Dadurch, dass der Positionsstent erfindungsgemäß als ein in ein Blutgefäß des Patienten einführbares, selbst expandierbares Bauteil ausgeführt ist, kann dieses vorab, d.h. vor der eigentlichen Implantation der am proximalen Ende des Herzklappenstents vorgesehenen Herzklappenprothese, eingesetzt werden. Denkbar hierbei wäre somit, dass der Positionsstent vorab in die Aorta eingebracht und dort optimal positioniert und fixiert wird, wobei anschließend der Herzklappenstent mit der Herzklappenprothese eingeführt und durch den dort bereits positionierten und fixierten Positionsstent in optimaler Weise eingesetzt wird.

Erfindungsgemäß sind sowohl der Herzklappenstent als auch der Positionsstent selbst expandierbar ausgeführt, was das jeweilige Einführen dieser Bauteile erleichtert. Dadurch, dass der Positionsstent, der die Aufgabe der Festlegung der Position für den Herzklappenstent bzw. die daran angeordnete Herzklappenprothese übernimmt, im Vergleich zu bisherigen selbst expandierbaren Herzklappenprothesen wesentlich kleiner ausführbar ist, wird die Manövrierbarkeit des Positionsstents deutlich erhöht, was letztendlich dazu führt, dass die Position, in welche der Positionsstent hinsichtlich der Herzklappe verankert wird, äußerst genau und an die jeweiligen Erfordernisse optimal angepasst gewählt werden kann. Dieser Vorteil der genauen Positionierung des relativ leicht zu manövrierenden und klein ausgeführten Positionsstents wird bei der anschließenden Implantation der Herzklappenprothese übernommen, da der Herzklappenstent, an dessen proximalen Ende die Herzklappenprothese vorgesehen ist, in der durch den (optimal positionierten) Positionsstent vorgegeben Position gehalten wird.

Im Hinblick auf das Einsetzen des Herzklappenstents ist erfindungsgemäß vorgesehen, dass der Herzklappenstent reversibel zusammen- und auseinanderfaltbar ausgeführt ist. Dabei ist es denkbar, dass der Herzklappenstent beispielsweise durch eine externe Manipulation zusammenfaltbar und mit Hilfe eines Explantations-Katheters entnehmbar ist. Dabei kann der Herzklappenstent in gefalteter Form in einer mit einem Positionstent-Katheter bzw. mit einem Explantations-Katheter verbindbaren Kartusche aufgenommen werden.

Vorteilhafte Weiterentwicklungen der erfindungsgemäßen Vorrichtung sind in den Unteransprüchen angegeben.

Damit der Herzklappenstent in optimaler Weise in ein Blutgefäß des Patienten eingeführt und dort in einer vorgegebenen Position hinsichtlich der Herzklappe positioniert werden kann, ist es erforderlich, dass der Positionsstent im zusammengefalteten Zustand möglichst klein ist, um somit eine optimale Navigation des Stents mit möglichst geringem Einfluss auf die Herzklappe zu ermöglichen. Dieses wird dadurch erreicht, dass die zu implantierende Herzklappenprothese nicht im Positionsstent sondern im Herzklappenstent angebracht ist. Ferner ist der Positionsstent so ausgeführt, dass sämtliche Teile des Stents im zusammengefalteten Zustand ein gewisses Maß einer radial nach außen wirkenden Vorspannung aufweisen, die nach Freigabe durch eine Kartusche die Selbstexpansion bewirken. Dann kann der Positionsstent mit der Kartusche mit Hilfe eines Positionsstent-Katheters beispielsweise durch eine Leistenarterie auf herkömmlichen Wege implantiert werden. Im Falle einer Fehlimplantation des Positionsstents, beispielsweise wenn die Position des Positionsstents in der Aorta des Patienten nicht exakt vorliegt, oder wenn aus anderen Gründen eine Explantation des Positionsstents erforderlich ist, ist vorgesehen, dass der Positionsstent von seinem expandierten Zustand wieder in den zusammengefalteten Zustand überführbar ist. Dieses erfolgt beispielsweise durch eine externe Manipulation mit Hilfe eines Implantatations-Katheters. Somit ist der Positionsstent vollkommen reversibel in den Katheter zurückziehbar, welches das vollständige Entfernen des Stents ermöglicht.

In vorteilhafter Weise der erfindungsgemäßen Vorrichtung zur transvaskulären Implantation und Befestigung von Herzklappenprothesen kann vorgesehen sein, dass der Positionsstent an seinem proximalen Ende eine Verankerung, insbesondere eine Verankerungsstütze, aufweist, wobei diese Verankerungsstütze derart ausgeführt ist, dass sich der Positionsstent in seinem expandierten Zustand selbstständig in einer vorgebbaren Position hinsichtlich der Herzklappe des Patienten positioniert und mit Hilfe der Verankerungsstütze gehalten wird. Dabei ist der Positionsstent so ausgebildet, dass die Verankerungsstütze in gefalteter Form in einer mit einem Katheter verbindbaren Kartusche aufgenommen ist. Die Verankerungsstütze sollte dabei so komprimiert werden, dass sie eine radial nach außen wirkende Vorspannung aufweist, die nach Freigabe durch eine Kartusche die Selbstexpansion bewirken. Durch die Ausbildung des Positionsstents, dass er sich in seinem expandierten Zustand selbstständig in eine vorgegebene Position hinsichtlich der Herzklappen des Patienten positioniert und dort mit Hilfe der Verankerungsstütze gehalten wird, ist es möglich, dass die Position des Positionsstents und damit die Position des Herzklappenstents vorab genau festlegbar ist, so dass Fehlimplantationen, wie sie bei den bekannten Lösungen möglich waren, ausgeschlossen werden können.

Um eine Selbstexpansion des Positionsstents zu erleichtern, kann in vorteilhafter Weise der Positionsstent ferner Vorspannelemente aufweisen, um den Positionsstent in seiner durch die Verankerung festgelegten Position in radialer Richtung vorzuspannen. Dabei sind auch die Vorspannelemente reversibel ausgeführt, so dass ihre Vorspannfunktion durch eine externe Manipulation aufgehoben werden kann, was das Zusammenfalten des Positionsstents und somit die Zurückziehbarkeit des Positionsstents in einen Katheter bzw. das vollständige Entfernen des Positionsstents ermöglicht.

In einer vorteilhaften Realisierung der zuletzt genannten Ausführungsformen ist vorgesehen, dass die Verankerungsstütze zumindest einen Stützbügel aufweist, der derart ausgeführt ist, dass er sich im expandierten Zustand des Positionsstents selbständig in die Taschen der Herzklappe des Patienten legt und somit die Ausrichtung des Positionsstents bezüglich der Herzklappe in axialer und horizontaler Richtung festlegt. Denkbar hierbei wäre beispielsweise, dass sich die an dem Positionsstent proximalseitig ausgebildeten Stützbügel während des Implantationsvorganges des Positionsstents selbstständig in die Taschen von Herzklappen des jeweiligen Patienten einsetzen, wobei die Taschen der Herzklappen ein Widerlager bilden, das der proximalen Einführbewegung entgegenwirkt, so dass die Verankerungsstütze mit dem Positionsstent lateral exakt positionierbar ist. Gleichzeitig wird gesichert, dass die Verankerungsstütze und der Positionsstent eine exakte Winkellage einnehmen können, da hierfür die Taschen während des Einführens quasi eine Führung für die Stützbügel darstellen. Erst nachdem die Stützbügel in die Taschen der Herzklappe des jeweiligen Patienten eingeführt worden sind und die letztendliche Position für den Positionsstent erreicht worden ist, wird der separat von dem Positionsstent ausgebildete Herzklappenstent mit Hilfe beispielsweise eines Herzklappen-Katheters implantiert. Der Herzklappenstent, der an seinem proximalen Ende die Herzklappenprothese aufweist, wird durch den bereits exakt positionierten und fixierten Positionsstent dann in optimaler Weise am günstigsten und besten Ort implantiert. Als weiterer Vorteil sei zu nennen, dass die Stützbügel des Positionsstents nach der Implantation des Positionsstents auf der Herzklappe des Patienten liegen. Dadurch, dass der Positionsstent relativ einfach ausgeführt ist, da er beispielsweise nicht die Herzklappenprothese aufweist, die an dem separat vom Positionsstent ausgebildeten Herzklappenstent vorgesehen ist, können die Bügel des Positionsstents einen relativ großen Radius aufweisen, welches eine geringere Verletzungsgefahr der Herzklappe bedeutet.

Die an einer Verankerungsstütze bzw. Verankerung vorhandenen Stützbügel sollten in proximaler Richtung konvex und bogenförmig gekrümmt sein, denn durch eine solch abgerundete Form können Verletzungen des Blutgefäßes am Herz vermieden sowie das Entfalten bei der Selbstexpansion erleichtert werden. Ebenfalls können die Stützbügel mit einer solchen Gestaltung entsprechend einfacher in die Taschen der alten Herzklappe eingeführt werden, ohne dass entsprechende Verletzungen am Gewebe oder dort vorhandenen Blutgefäßen zu verzeichnen sind.

An den Verankerungsstützen können aber auch zusätzliche Stabilisierungsbügel vorhanden sein, mit denen eine Erhöhung der Festigkeit nach der Selbstexpansion der verankerten Verankerungsstütze erreichbar ist. Solche Stabilisierungsbügel können vorteilhaft sein, da für die Ausnutzung des erforderlichen Selbstexpansionseffekts an einer Verankerungsstütze für eine sichere Fixierung der Verankerungsstütze mit dem Positionsstent, unter Berücksichtigung der Tatsache, dass während der Phase des Einführens einer innerhalb einer Kartusche gefalteten Verankerungsstütze möglichst kleine Volumina erreicht werden müssen, kleine Querschnitte für die jeweiligen Bügel eingehalten werden müssen.

Sämtliche Bügel einer Verankerungsstütze sollten dabei so angeordnet, gestaltet und dimensioniert sein, dass die nacheinander erfolgende Freigabe von Stützbügeln und den anderen Bügeln mit gegebenenfalls weiteren an einer Verankerungsstütze vorhandenen Elementen durch entsprechende Manipulation von Kartusche und/oder Katheter erreicht werden können. Dabei sollte selbstverständlich auch die Gestaltung der Kartusche oder mindestens eines Teiles einer Kartusche berücksichtigt sein.

Entsprechend der Anatomie sollten an der Verankerungsstütze drei in jeweils gleichen Winkelabständen zueinander angeordnete Stützbügel vorhanden sein. Es besteht aber auch die Möglichkeit, dass die an einer Verankerungsstütze vorhandenen Stützbügel jeweils in einem Winkelversatz zueinander angeordnet sind. In diesem Fall sind dann im implantierten Zustand die Stützbügel mit ihren proximalen Teilen in die Tasche einer alten Herzklappe eingeführt und die alte Herzklappe kann dann klemmend mit Stützbügeln gehalten werden.

Die Stabilität eines implantierten und befestigten Positionsstents kann optimal mittels mindestens eines Ringträgers, der ein Element an einer Verankerungsstütze sein kann, erhöht werden. So besteht die Möglichkeit, die an einer Verankerungsstütze vorhandenen unterschiedlichen Bügel, bevorzugt an deren Fußpunkten mit einem solchen Ringträger zu verbinden. Dabei ist es nicht zwingend erforderlich, eine Verbindung zwischen Ringträger und sämtlichen Bügeln einer Verankerungsstütze vorzusehen.

Nachdem der Positionsstent an dem Herzen positioniert und dort mit Hilfe der Verankerungsstütze gehalten wird, wird der Herzklappenstent eingeführt. Dabei ist in vorteilhafter Weise vorgesehen, dass der Herzklappenstent derart ausgelegt ist, dass die Herzklappenprothese in ihrem expandierten Zustand die Herzklappe des Patienten gegen die Aortenwand drückt, wobei die zumindest eine Verankerung des Positionsstents zwischen der Aortenwand und der mit der Herzklappenprothese aufgeklappten Herzklappe liegt.

Um zu erreichen, dass der Herzklappenstent mit Hilfe des Positionsstents in einer durch den Positionsstent vorgebbaren Position hinsichtlich der Herzklappe des Patienten gehalten werden kann, weist der Positionsstent an seinem distalen Ende zumindest ein Einrastelement auf. Der Herzklappenstent sollte dabei an seinem distalen Ende ein entsprechend komplementär ausgeführtes Halteelement aufweisen, wobei im expandierten Zustand des Positionsstents und im vollständig expandierten Zustand des Herzklappenstents das zumindest eine Halteelement mit dem zumindest einen Einrastelement des Positionsstents eine kraftschlüssige Verbindung bilden. Damit kann erreicht werden, dass die Herzklappenprothese in der durch den Positionsstent vorgegebenen Position in dem Herzkranzgefäß positioniert und dort mit dem Positionsstent gehalten wird. Denkbar hierbei wäre es, dass am Herzklappenstent Einrastbügel vorgesehen sind. Dabei zählen die Einrastbügel zu Elementen des Herzklappenstents, die erst dann zu ihrer Expansion freigegeben werden, wenn der Herzklappenstent mit Hilfe des bereits implantierten Positionsstents positionsrichtig in ihren Implantationsort an einer Herzklappe des Patienten eingeführt worden sind. Beim Entfalten der Einrastbügel des Herzklappenstents greifen diese in die Einrastelemente des Positionsstents ein und halten so den Herzklappenstent in der durch den Positionsstent vorgegebenen Position. Gleichzeitig gelangen dann Teile der alten Herzklappe des jeweiligen Patienten zwischen jeweils einen Verankerungsbügel des Positionsstents und der expandierten Herzklappenprothese, so dass die jeweiligen Teile der alten Herzklappe zwischen diesen Elementen nach erfolgter Entfaltung der Herzklappenprothese klemmend gehalten werden, ähnlich wie es mit einem Papier zwischen den Bügeln einer Büroklammer der Fall ist.

Der Herzklappenstent ist insbesondere derart ausgeführt, dass er seinen vollständig expandierten Zustand, bei dem sowohl die Herzklappenprothese als auch das Halteelement freigegeben sind, erst dann einnimmt, wenn der Herzklappenstent in der durch den Positionsstent vorgegebenen Position liegt.

Der Herzklappenstent ist, wie auch der Positionsstent, in vorteilhafter Weise reversibel zusammen- und auseinanderfaltbar ausgeführt, wobei im zusammengefalteten Zustand die kraftschlüssige Verbindung mit dem Positionsstent gelöst ist. Damit kann erreicht werden, dass die an dem Herzklappenstent vorgesehene Herzklappenprothese beispielsweise bei einer fehlerhaften Implantation wieder explantiert werden kann, ohne dass hierzu auch der Positionsstent entfernt werden muss.

Um eine Explantation des Herzklappenstents zu erleichtern, können am distalen Ende des Herzklappenstents Explantations-Elemente vorgesehen sein, die mit dem Herzklappenstent derart zusammenwirken, dass beispielsweise durch eine externe Manipulation der Explantations-Elemente die kraftschlüssige Verbindung zwischen dem Herzklappenstent und dem Positionsstent gelöst und der Herzklappenstent zusammengefaltet wird. In einer vorteilhaften Realisierung der Explantations-Elemente ist vorgesehen, dass sie mit Hilfe beispielsweise eines Explantations-Katheters aufgreifbar sind, wobei durch ein Einziehen der Explantations-Elemente in den Explantations-Katheter die kraftschlüssige Verbindung zwischen dem Herzklappenstent und dem Positionsstent gelöst und der Herzklappenstent zusammengefaltet wird.

In vorteilhafter Weise ist der Herzklappenstent im gefalteten Zustand in einer mit einem Herzklappenstent-Katheter und/oder Explantations-Katheter verbindbaren Kartusche aufgenommen, wobei durch eine vorgebare Bewegung der Kartusche der Herzklappenstent freigebbar ist. Im Einzelnen ist in vorteilhafter Weise dabei vorgesehen, dass durch eine vorgebbare erste Bewegung der Kartusche lediglich die Herzklappenprothese zum Entfalten dieser freigebbar ist, wobei durch mindestens eine zweite nachfolgende Bewegung der Kartusche bzw. des Katheters das Halteelement des Herzklappenstents freigegeben ist.

Insbesondere für die nachfolgenden mittels Kartusche und Katheter durchzuführenden Bewegungen, die zur sequentiellen Freigabe der einzelnen Elemente des Herzklappenstents führen, kann es vorteilhaft sein, eine mehrteilige Kartusche einzusetzen, wobei mindestens zwei einzelne Teile jeweils relativ zueinander bewegt werden können. So können beispielsweise die für die Selbstexpansion durchzuführenden Bewegungen einer Kartusche oder einzelner Teile einer Kartusche ein proximales und/oder distales Verschieben sein, das in mehreren nacheinander durchzuführenden Stufen erfolgen kann, wobei jeweils unterschiedliche Wege zurückgelegt werden, um die entsprechenden Teile zum jeweiligen Zeitpunkt einer Implantation für deren Expansion nacheinander freizugeben.

So kann beispielsweise die erste Bewegung ein distales Zurückziehen der Kartusche oder eines Teiles einer Kartusche sein. In diesem Fall kann dann, falls dies erforderlich sein sollte, um eine Fehlimplantation zu vermeiden, eine proximale Bewegung der Kartusche oder eines Teils einer Kartusche dazu benutzt werden, um die bereits entfalteten radial nach außen mit einer Vorspannkraft wirkenden Halteelemente bzw. die Herzklappenprothese des Herzklappenstents wieder zusammenzufalten und im inneren der Kartusche aufzunehmen, so dass eine Entfernung der Vorrichtung aus dem Patienten möglich ist. Als Betätigungselemente für eine Manipulation und dementsprechend eine Verschiebungsbewegung der Kartusche oder einzelner Teile der Kartusche können Baudenzüge oder flexible Schubrohre eingesetzt werden, die durch das Innere des Katheters zur Kartusche oder zu einem Teil der Kartusche geführt sind. Solche Betätigungselemente können aber auch an Befestigungselementen, beispielsweise Ösen, die an der Verankerungsstütze vorhanden sind, angreifen.

Bei der erfindungsgemäßen Lösung besteht somit die Möglichkeit, auch eine nicht zum Erfolg führende Implantation von Herzklappenprothesen abzubrechen und die Vorrichtung durch Zurückziehen des Katheters wieder zu entfernen, wobei hierfür der bereits entfaltete Herzklappenstent wieder zusammengefaltet und in einer Kartusche oder ein Teil einer Kartusche rückgeführt werden kann.

In einer vorteilhaften Weiterentwicklung der erfindungsgemäßen Vorrichtung ist vorgesehen, dass der Positionsstent ferner Verankerungselemente, insbesondere Haken, aufweist, um den Positionsstent in seiner vorgebbaren Position am Herzen zu verankern. Möglich wäre auch, dass zusätzlich oder alternativ zum Positionsstent der Herzklappenstent Verankerungselemente, wie beispielsweise Haken, aufweist, um den Herzklappenstent in der mit dem Positionsstent vorgegebenen Position in der Aorta zu verankern. Beide Lösungen dienen letztendlich zum sicheren Fixieren der implantierten Herzklappenprothese in ihrem durch den Positionsstent vorgegebenen Implantationsort.

Um die räumliche Orientierung beim Einsetzen des Positionsstents zu erleichtern, können Marker, insbesondere Röntgenmarker, am Positionsstent vorgesehen sein. Selbstverständlich sind aber auch andere Lösungen denkbar. Beispielsweise kann das Einführen des Positionsstents auch mit Hilfe einer Röntgendurchleuchtung (Herzkatheterlabor = HKL) oder mit Hilfe von Ultraschall (transösophageales Echokardiogramm = TEE) überwacht und gesteuert werden.

Der Positionsstent und/oder der Herzklappenstent kann ferner Führungsmittel aufweisen, die derart ausgelegt sind, dass der Herzklappenstent im expandierten Positionsstent selbstständig in die durch den Positionsstent vorgegebenen Position geführt wird. Denkbar wäre hierbei, dass die Führungsmittel als sich zum distalen Ende des Positionsstents bzw. des Herzklappenstents hin verjüngende Elemente ausgeführt sind, so dass eine selbstständige Justage des Herzklappenstents im Positionsstent und somit in der durch den Positionsstent vorgegebenen Position erfolgen kann.

Die erfindungsgemäße Vorrichtung kann auch gemeinsam mit einem Ballonkatheter eingesetzt werden. Mit dem Ballonkatheter kann die alte Herzklappe vor der Selbstexpansion der Verankerungsstütze weggedrückt werden.

Im Folgenden werden bevorzugte Ausführungsformen der erfindungsgemäßen Vorrichtung zur Implantation und Befestigung von Herzklappenprothesen anhand der beigefügten Zeichnungen näher erläutert.

Es zeigen:
- Fig. 1:: eine bevorzugte Ausführungsform eines Positionsstents der erfindungsgemäßen Vorrichtung im eingesetzten und expandierten Zustand;
- Fig. 2A:: eine bevorzugte Ausführungsform eines Herzklappenstents der erfindungsgemäßen Vorrichtung im expandierten Zustand;
- Fig. 2B:: den in Fig. 2A gezeigten Herzklappenstent im implantierten Zustand;
- Fig. 3A, B:: jeweils eine schematische Darstellung zur Erläuterung des Explantationsvorganges einer bevorzugten Ausführungsform des Herzklappenstents; und

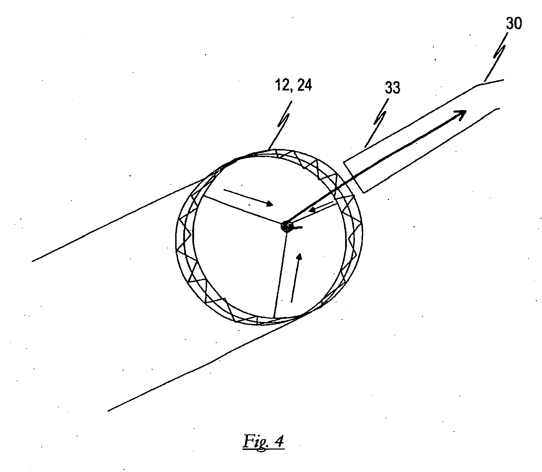

- Fig. 4:: eine detaillierte Darstellung der am Herzklappenstent bzw. Positionsstent vorgesehenen Explantations-Elemente sowie die Funktionsweise hiervon.

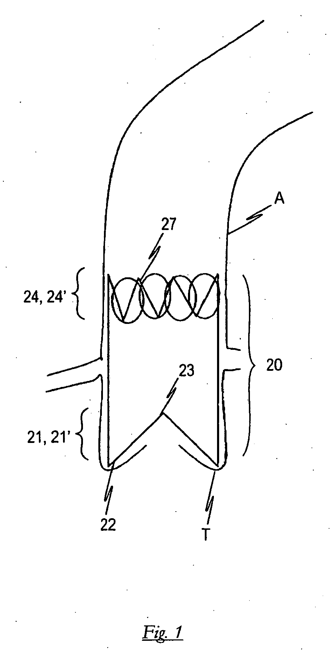

Fig. 1 zeigt eine bevorzugte Ausführungsform eines Positionsstents 20 der erfindungsgemäßen Vorrichtung im eingesetzten Zustand. Der Positionsstent 20 befindet sich in der gezeigten Ausführungsform in seinem expandierten Zustand. Wie dargestellt, weist der Positionsstent 20 an seinem proximalen Ende ein Verankerungs-Segment 21' mit Verankerungsstützen 21 auf. Die Verankerungsstützen 21 sind dabei so ausgeführt, dass sie sich in die Taschen T der alten Herzklappe bezüglich der axialen Rotation wie auch der horizontalen Position selbst optimieren. Hierzu wird der Positionsstent 20 über die Verankerungsstützen 21 in den Taschen T der alten Herzklappe abgestützt. Die Verankerungsstützen 21 selber sind über Stege 22 mit einem Andock-Segment 23 verbunden. Das Andock-Segment 24' des Positionsstents 20, das an seinem distalen Ende vorgesehen ist, weist eine Vielzahl von Einrastelementen 24 auf, mit denen ein zu implantierender Herzklappenstent (in Fig. 1 nicht explizit gezeigt) fixiert wird.

Der Positionsstent 20 ist als ein selbst expandierendes Bauteil ausgeführt. Aufgrund des einfachen Aufbaus des Positionsstents 20, der im wesentlichen lediglich nur aus dem Verankerungs-Segment 21', dem Andock-Segment 24' sowie den Stegen 22 besteht, weist der Positionsstent 20 in seinem zusammengefalteten Zustand äußerst geringe Dimensionen auf. Beim Einsetzen des Positionsstent 20, beispielsweise mit Hilfe eines Positionsstent-Katheters, lässt der sich der Positionsstent 20 somit sehr gut in der Aorta A manövrieren. Nachdem der Positionsstent 20 in die Aorta A eingebracht wurde, wird dieser auseinandergefaltet, was beispielsweise durch eine externe Manipulation des Positionsstent-Katheters erfolgt. Die Verankerungsstützen 21 des expandierten Positionsstents 20 legen sich selbstständig in die Taschen T der Herzklappe des Patienten, wodurch die Ausrichtung des Positionsstents 20 bezüglich der Herzklappe in axialer und horizontaler Richtung festgelegt wird. Damit das Auseinanderfalten des Positionsstents 20 selbstständig erfolgt, können (optional) geeignete Vorspannelemente vorgesehen sein. In der dargestellten Ausführungsform sind Vorspannelemente in der Gestalt der Verankerungsstützen 21 ausgeführt.

Nachdem der Positionsstent 20 in die Aorta A eingeführt und dort, wie zuvor beschrieben, positioniert und fixiert wurde, wird in den Positionsstent 20 ein Herzklappenstent 10 eingebracht, welcher in seinem proximalen Ende eine Herzklappenprothese 11 beinhaltet. Diese wird nach Freisetzung entfaltet und drückt die alte Klappe dabei gegen die Aortenwand bzw. den Positionsstent 20.

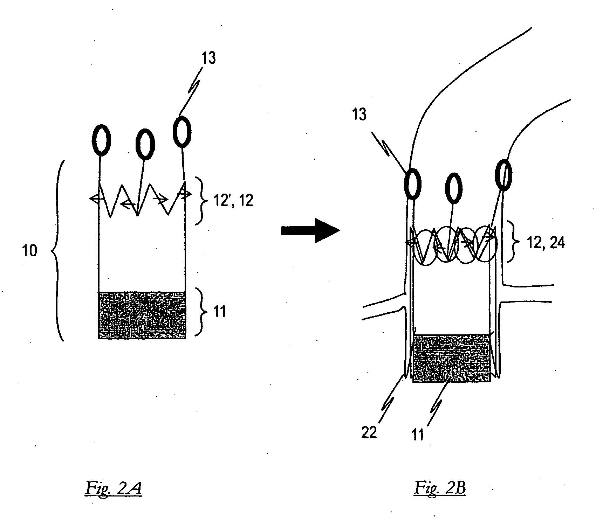

In Fig. 2A ist ein Herzklappenstent 10 im expandierten Zustand gezeigt. Wie dargestellt weist der Herzklappenstent 10 an seinem proximalen Ende die Herzklappenprothese 11 und an seinem distalen Ende ein Verankerungs-Segment 12' auf, das zumindest ein Halteelement 12 aufweist.

In Fig. 2B ist dargestellt, wie der Herzklappenstent 10 in dem bereits positionierten und fixierten Positionsstent 20 gehalten wird. Der Herzklappenstent 10 wird über Führungselemente 17, 27 im Positionsstent 20 so bezüglich axialer Rotation und Position geführt, dass die neue Herzklappe optimal zu liegen kommt. Nachdem dies geschehen ist, wird durch weiteres Freigeben des Herzklappenstents 10 dessen Verankerungs-Segment 12' im Andock-Segment 24' des Positionsstents 20 eingebracht. Das Verankerungs-Segment 12' weist Haltelemente 12 auf, die mit den Einrastelementen 24 des Positionsstents 10 eine kraftschlüssige Verbindung bilden, um die Herzklappenprothese 11 in der durch den Positionsstent 20 vorgegebenen Position in dem Herzkranzgefäß zu positionieren und dort über den Positionsstent 20 zu halten.

Im Gegensatz zu einem herkömmlichen Herzklappenstent weist der Herzklappenstent 10 der vorliegenden Vorrichtung keine Haltebügel auf, die hinter die alte Herzklappe eingreifen, sondern Einrastbügel in der Gestalt von Halteelemente 12 im Verankerungs-Segment 12 des Herzklappenstents 10. Diese Einrastbügel treten in Wechselwirkung mit den Einrastelementen 24, die im Andock-Segment 24' des Positionsstents 20 vorgesehen sind. Der Vorteil hiervon ist, dass der Herzklappenstent 10 in dem Positionsstent 20 auswechselbar verankert ist. Der Herzklappenstent 10 rutscht durch seine Selbstfindung, die durch Führungsmittel 17, 27 bewirkt wird, selbstständig in den Positionsstent 20 hinein und kann nicht weiter rutschen. Die Führungsmittel 17, 27 sind als sich zum distalen Ende des Positionsstents 20 und/oder des Herzklappenstents 10 hirn verjüngende Elemente ausgeführt. Durch die besondere Ausgestaltung der Einrastelemente 23 des Positionsstent 20 und der Halteelemente 12 des Herzklappenstents 10 in der Gestalt von Zick-Zack-Bügeln (Z-Periodenbügel) kann insbesondere eine feinere Winkelpositionierung des Herzklappenstents 10 erfolgen. Sowohl der Positionsstent 20 als auch der Herzklappenstent 10 können aus einzelnen Segmenten aufgebaut sein, wobei die einzelnen Segmente zueinander verdreht werden können. Dieses erhöht die Flexibilität, mit denen die beiden Stents in der Aorta eingesetzt werden können. Insbesondere ist es möglich, eine feinere Winkelpositionierung des Herzklappenstents 10 durchzuführen. So ist es beispielsweise denkbar, dass die Herzklappenprothese 11 von dem Arzt wahlweise verdreht eingesetzt werden kann. Auch im Hinblick auf das Zusammenfalten des Herzklappenstents und des Positionsstents ist der segmentförmige Aufbau von Vorteil, da die Stents segmentförmig verdreht gefaltet in einem Katheter verstaut werden können.

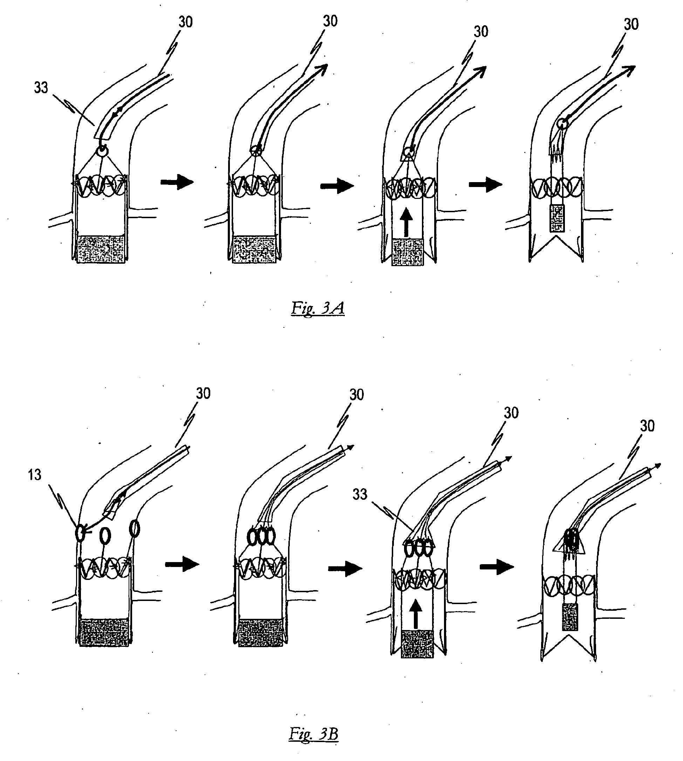

Fig. 3A zeigt eine schematische Darstellung, wie der Herzklappenstent 10 in den bereits positionierten und implantierten Positionsstent 20 explantiert werden kann. Im Falle einer Klappendysfunktion kann die zuvor beschriebene mechanisch stabile Verbindung zwischen dem Positionsstent 20 und dem Herzklappenstent 10 durch ein externe Manipulation wieder gelöst werden. Dieses geschieht beispielsweise durch ein Aufgreifen von Explantations-Elementen 13 mit einem Katheter 30 und einer daran befestigten Kartusche 33. Nach Einziehen der Explantations-Elemente 13 in den variabel trichterförmig ausgeführten Explantations-Katheter 30 wird der Herzklappenstent 10 in diesen hineingezogen und kann so durch einen neuen ersetzt werden. Der Positionsstent 20 verbleibt als Markierung und Verankerungsbasis für einen neuen Herzklappenstent 10. Selbstverständlich ist aber auch der Positionsstent 20 auf ähnliche Weise explantierbar.

Das Aridock-Segment 23' des Positionsstents kann Ösen oder Noppen aufweisen, an denen der Explantations-Katheter 30 zu befestigen ist, um eine solche Explantation zu bewirken. Die Befestigung an Ösen ist über vorzugsweise drei bis sechs Ösen und drei bis sechs Schlaufen möglich, welche später aus den Ösen gezogen werden. Insbesondere ist der Positionsstent 20, wie auch der Herzklappenstent 10 vollkommen reversibel in den Katheter zurückziehbar, welches das vollständige Entfernen des Positionsstents und/oder des Herzklappenstents ermöglicht.

Das Lösen der mechanisch stabilen Verbindung zwischen dem Positionsstent 20 und dem Herzklappenstent 10 durch ein externe Manipulation, beispielsweise im Fall einer Klappendysfunktion, ist möglich, da der zuvor implantierte Herzklappenstent 10 eine dafür geeignete Rückholkonstruktion aufweist. Diese besteht aus mehreren Verbindungsstegen, welche vom oberen äußeren Ende des Stents nach medial in das Gefäßlumen ragen und sich dort in einer Verankerungsvorrichtung (Öse, Haken, Knopf etc.) vereinen. Wird diese Verankerungsvorrichtung nun durch den Rückholkatheterdraht des Katheters 30 gefasst, kann so der Herzklappenstent 10 in seinem distalen Anteil lumenwärts zusammengezogen und in einen Katheterschlauch 33 gezogen werden. Es bietet sich dann wieder die Möglichkeit, bei verbleibendem Positionsstent 20 diesen als Markierung und Verankerungsbasis für einen neuen Herzklappenstent 10 zu nutzen.

Der Positionsstent 20 ist aus einem festen Geflecht (Draht, Polymer etc.) hergestellt, oder wird durch ein Laserschnittverfahren erzeugt. Als geeignete Materialien des Positionsstents kommt NiTi, Edelstahl oder bio-kompatible Kunststoffe in Frage. Zur räumlichen Orientierung können des weiteren Röntgenmarken auf dem Positionsstent 20 gesetzt werden.

## Patentansprüche

1. Vorrichtung zur transvaskulären Implantation und Befestigung von Herzklappenprothesen, mit einem durch ein Blutgefäß eines Patienten einführbaren, selbstexpandierbaren Herzklappenstent (10), der an seinem proximalen Ende eine Herzklappenprothese (11) aufweist, wobei
die Vorrichtung ferner einen bis in die Taschen der erkrankten Herzklappe eines Patienten einführbaren, selbstexpandierbaren Positionsstent (20) aufweist, der von dem Herzklappenstent (10) separat einzuführen ist, wobei der Positionsstent (20) und der Herzklappenstent (10) so ausgeführt sind, dass der Herzklappenstent (10) in seinem expandierten Zustand mit Hilfe des expandierten Positionsstents (20) in einer durch den Positiontsstent (20) vorgebbaren Position hinsichtlich der Herzklappe im ventrikulären Ausflusstrakt und herznahen Gefäßen des Patienten fixierbar ist,
**dadurch gekennzeichnet, dass**
der Herzklappenstent (20) reversibel zusammen- und auseinanderfaltbar ausgeführt ist, wobei der Herzklappenstent (20) durch eine externe Manipulation zusammenfaltbar und mit Hilfe eines Explantations-Katheters (30) aus dem Positionsstent entnehmbar ist.

2. Vorrichtung nach Anspruch 1, wobei
der Positionsstent (20) an seinem proximalen Ende eine Verankerung (21), insbesondere Verankerungsstütze, aufweist, wobei die Verankerungsstütze (21) derart ausgeführt ist, dass der Positionsstent (20) in seinem expandierten Zustand selbständig in einer vorgebbaren Position hinsichtlich der Herzklappe des Patienten positionierbar und dort mit Hilfe der Verankerungsstütze (21) verankerbar ist.

3. Vorrichtung nach Anspruch 2, wobei
die Verankerungsstütze (21) zumindest einen Stützbügel aufweist, der derart ausgeführt ist, dass er im expandierten Zustand des Positionsstents (20) selbständig in die Taschen der Herzklappe des Patienten legbar ist zum Festlegen der Ausrichtung des Positionsstents (20) bezüglich der Herzklappe in axialer und horizontaler Richtung.

4. Vorrichtung nach Anspruch 2 oder 3, wobei
der Positionsstent (20) ferner Vorspannelemente (23) aufweist zum Vorspannen des Positionsstents (20) in seiner durch die Verankerung (21) festgelegten Position in radialer Richtung.

5. Vorrichtung nach einem der Ansprüche 2 bis 4, wobei
der Herzklappenstent (10) ausgebildet ist zum Drücken der Herzklappe des Patienten gegen die Gefäßwand mit Hilfe der Herzklappenprothese (11) in ihrem expandierten Zustand, wobei die zumindest eine Verankerung (21) des Positionsstents (20) zwischen der Gefäßwand und der mit der Herzklappenprothese (11) aufgeklappten Herzklappe liegt.

6. Vorrichtung nach einem der vorhergehenden Ansprüche, wobei
der Positionsstent (20) zumindest ein Einrastelement (24) und der Herzklappenstent (10) zumindest ein Halteelement (12) aufweist zum Bilden einer kraftschlüssigen Verbindung zwischen dem zumindest einen Halteelement (12) und dem zumindest einen Einrastelement (24) des Positionsstents (20) im expandierten Zustand des Positionsstents (20) und im vollständig expandierten Zustand des Herzklappenstents (10), um die Herzklappenprothese (11) in der durch den Positionsstent (20) vorgegeben Position zu positionieren und dort mit dem Positionsstent (20) zu halten.

7. Vorrichtung nach Anspruch 6, wobei
der Herzklappenstent (10) reversibel zusammen- und auseinanderfaltbar ausgeführt ist, wobei das zumindest eine Einrastelement (24) und das zumindest eine Halteelement (12) so ausgebildet sind, dass im zusammengefalteten Zustand die kraftschlüssige Verbindung mit dem Positionsstent (20) gelöst ist.

8. Vorrichtung nach einem der vorhergehenden Ansprüche, wobei am Herzklappenstent (10) ferner Explantations-Elemente (13) vorgesehen sind, wobei die Explantations-Elemente (13) und der Herzklappenstent (10) so ausgebildet sind, dass durch eine externe Manipulation der Explantations-Elemente (13) die kraftschlüssige Verbindung zwischen dem Herzklappenstent (10) und dem Positionsstent (20) gelöst und der Herzklappenstent (10) zusammengefaltet wird.

9. Vorrichtung nach Anspruch 8, wobei
die Explantations-Elemente (13) derart ausgeführt sind, dass sie mit Hilfe eines Katheters (30) aufgreifbar sind, wobei die Explantations-Elemente (13) derart ausgeführt sind, dass durch ein Einziehen der Explantations-Elemente (13) in den Katheters (30) die kraftschlüssige Verbindung zwischen dem Herzklappenstent (10) und dem Positionsstent (20) lösbar und der Herzklappenstent (10) zusammenfaltbar ist.

10. Vorrichtung nach einem der Ansprüche 7 bis 9, wobei
der Herzklappenstent (10) im gefalteten Zustand in einer mit einem Katheter (30) verbindbaren Kartusche (33) aufgenommen werden kann wobei durch eine vorgebbare Bewegung der Kartusche (33) der Herzklappenstent (10) freigebbar ist.

11. Vorrichtung nach Anspruch 10, wobei
durch eine vorgebbare erste Bewegung der Kartusche (33) lediglich die Herzklappenprothese (11) zum Entfalten der Herzklappenprothese (11) freigebbar ist, und wobei durch mindestens eine zweite nachfolgende Bewegung der Kartusche (33) und/oder des Katheters (30) das Halteelement (12) freigebbar ist.

12. Vorrichtung nach einem der vorhergehenden Ansprüche, wobei
der Positionsstent (20) ferner Verankerungselemente, insbesondere Haken, aufweist, um den Positionsstent (20) in seiner vorgebbaren Position zu verankern.

13. Vorrichtung nach einem der vorhergehenden Ansprüche, wobei
der Herzklappenstent (10) ferner Verankerungselemente, insbesondere Haken, aufweist, um den Herzklappenstent (10) in der mit dem Positionsstent (20) vorgegebenen Position zu verankern.

14. Vorrichtung nach einem der vorhergehenden Ansprüche, wobei
der Positionsstent (20) ferner Marker, insbesondere Röntgenmarker, aufweist, um die räumliche Orientierung beim Einsetzen des Positionsstents (20) zu erleichtern.

15. Vorrichtung nach einem der vorhergehenden Ansprüche, wobei
der Herzklappenstent (10) derart ausgeführt ist, dass sein vollständig expandierter Zustand, bei dem sowohl die Herzklappenprothese (11) als auch das Halteelement (12) freigegeben sind, erst dann einnehmbar ist, wenn der Herzklappenstent (10) in der durch den Positionsstent (20) vorgegeben Position liegt.

16. Vorrichtung nach einem der vorhergehenden Ansprüche, wobei
der Positionsstent (20) und/oder der Herzklappenstent (10) ferner Führungsmittel (27, 17) aufweisen zum selbstständigen Führen des Herzklappenstents (10) in den expandierten Positionsstent (20) in die durch den Positionsstent (20) vorgebbare Position.

17. Vorrichtung nach Anspruch 16, wobei
die Führungsmittel (27, 17) als sich zum distalen Ende des Positionsstents (20) und/oder des Herzklappenstents (10) hin verjüngende Elemente ausgeführt sind zur selbstständigen Justage des Herzklappenstents (10) im Positionsstent (20) und somit in der durch den Positionsstent (20) vorgegebenen Position.

## Claims

1. Device for the transvascular implantation and fixing of heart valve prostheses, comprising a self-expandable heart valve stent (10) introducible through a blood vessel of a patient, which has a heart valve prosthesis (11) at its proximal end, wherein the device further comprises a self-expandable position stent (20) introducible as far as the pockets of the diseased heart valve of a patient, which is to be introduced separately from the heart valve stent (10), wherein the position stent (20) and the heart valve stent (10) are configured such that the heart valve stent (10) is fixable in its expanded state by means of the expanded position stent (20) in the ventricular outflow tract and vessels close to the patient's heart in a position relative to the heart valve, which is predefinable by the position stent (20),
**characterized in that**
the heart valve stent (20) is configured to be reversibly foldable and unfoldable, wherein the heart valve stent (20) can be folded together by an external manipulation and removed from the position stent by means of an explantation catheter (30).

2. Device according to claim 1, wherein the position stent (20) comprises an anchor (21) at its proximal end, specifically an anchoring support, wherein the anchoring support (21) is configured such that the position stent (20) is self-positionable in its expanded state in a predefinable position relative to the heart valve of the patient, in which it can be anchored by means of the anchoring support (21).

3. Device according to claim 2, wherein the anchoring support (21) comprises at least one support bracket which is configured to independently position itself in the pockets of the heart valve of the patient in the expanded state of the positioning stent (20) for fixing the orientation of the position stent (20) relative to the heart valve in the axial and horizontal direction.

4. Device according to claim 2 or 3, wherein the position stent (20) further comprises pretensioning elements (23) for pretensioning the position stent (20) in its position fixed by the anchor (21) in the radial direction.

5. Device according to one of claims 2 to 4, wherein the heart valve stent (10) is configured to press the heart valve of the patient against the vascular wall by means of the heart valve prosthesis (11) in its expanded state, wherein the at least one anchor (21) of the position stent (20) is positioned between the vascular wall and the heart valve expanded by the heart valve prosthesis (11).

6. Device according to one of the preceding claims, wherein the position stent (20) comprises at least one engaging element (24) and the heart valve stent (10) comprises at least one holding element (12) for forming a non-positive connection between the at least one holding element (12) and the at least one engaging element (24) of the position stent (20) in the expanded state of the position stent (20) and in the fully expanded state of the heart valve stent (10) so as to position the heart valve prosthesis (11) in the position predefined by the position stent (20) and hold it there by means of the position stent (20).

7. Device according to claim 6, wherein the heart valve stent (10) is configured to be reversibly foldable and unfoldable, wherein the at least one engaging element (24) and the at least one holding element (12) are configured such that the non-positive connection with the position stent (20) is disengaged in the folded state.

8. Device according to one of the preceding claims, wherein further explantation elements (13) are provided on the heart valve stent (10), wherein the explantation elements (13) and the heart valve stent (10) are configured such that by an external manipulation of the explantation elements (13) the non-positive connection between the heart valve stent (10) and the position stent (20) is disengaged and the heart valve stent (10) is folded together.

9. Device according to claim 8, wherein the explantation elements (13) are configured such that they are engageable by means of a catheter (30), wherein the explantation elements (13) are configured such that by pulling the explantation elements (13) into the catheter (30) the non-positive connection between the heart valve stent (10) and the position stent (20) can be disengaged and the heart valve stent (10) can be folded together.

10. Device according to one of claims 7 to 9, wherein the heart valve stent (10) can be received in the folded state in a cartridge (33) connectable to a catheter (30), wherein the heart valve stent (10) can be released by a predefinable motion of the cartridge (33).

11. Device according to claim 10, wherein merely the heart valve prosthesis (11) is releasable by a predefinable first motion of the cartridge (33) for unfolding the heart valve prosthesis (11), and wherein the holding element (12) is releasable by at least one second subsequent motion of the cartridge (33) and/or the catheter (30).

12. Device according to one of the preceding claims, wherein the position stent (20) further comprises anchoring elements, specifically hooks, so as to anchor the position stent (20) in its predefinable position.

13. Device according to one of the preceding claims, wherein the heart valve stent (10) further comprises anchoring elements, specifically hooks, so as to anchor the heart valve stent (10) in the position predefined by the position stent (20).

14. Device according to one of the preceding claims, wherein the position stent (20) further comprises markers, specifically X-ray markers, so as to facilitate the spatial orientation when the position stent (20) is inserted.

15. Device according to one of the preceding claims, wherein the heart valve stent (10) is configured such that its fully expanded state, in which both the heart valve prosthesis (11) and the holding element (12) are released, can only then be assumed when heart valve stent (10) is positioned in the position predefined by the position stent (20).

16. Device according to one of the preceding claims, wherein the position stent (20) and/or the heart valve stent (10) further comprise guide means (27, 17) for independently guiding the heart valve stent (10) into the expanded position stent (20) into the position predefinable by the position stent (20).

17. Device according to claim 16, wherein the guide means (27, 17) are configured as elements tapering to the distal end of the position stent (20) and/or the heart valve stent (10) for the self-adjustment of the heart valve stent (10) in the position stent (20) and thus in the position predefined by the position stent (20).

## Revendications

1. Dispositif pour l'implantation transvasculaire et la fixation de prothèses de valvules cardiaques, comprenant un stent de valvule cardiaque (10) autoexpansible susceptible d'être introduit via un vaisseau sanguin d'un patient et qui comporte une prothèse de valvule cardiaque (11) à son extrémité proximale, dans lequel
le dispositif comprend en outre un stent de positionnement (20) autoexpansible susceptible d'être introduit jusque dans les cuspides de la valvule cardiaque malade d'un patient, qui doit être introduit séparément du système de valvule cardiaque (10), le stent de positionnement (20) et le stent de valvule cardiaque (10) étant réalisés de telle façon que, dans son état en expansion, le stent de valvule cardiaque (10) est susceptible d'être fixé dans la sortie ventriculaire et les vaisseaux du patient proches du coeur, à l'aide du stent de positionnement (20) en expansion dans une position prédéterminée par le stent de positionnement (20) par rapport à la valvule cardiaque,
**caractérisé en ce que**
le stent de valvule cardiaque (20) est réalisé de manière à pouvoir être replié et déployé de façon réversible, et le stent de valvule cardiaque (20) est susceptible d'être replié par une manipulation externe et d'être enlevé hors du stent de positionnement à l'aide d'un cathéter d'explantation (30).

2. Dispositif selon la revendication 1, dans lequel
le stent de positionnement (20) comprend à son extrémité proximale un ancrage (21), en particulier un soutien d'ancrage, et que le soutien d'an-crage (21) est réalisé de telle manière que, dans son état en expansion, le stent de positionnement (20) peut être positionné automatiquement dans une position prédéterminée par rapport à la valvule cardiaque du patient et y être ancré à l'aide du soutien d'ancrage (21).

3. Dispositif selon la revendication 2,
dans lequel le soutien d'ancrage (21) comprend au moins un arceau de soutien qui est réalisé de telle façon que, dans l'état en expansion du stent de positionnement (20), il peut être posé automatiquement dans les cuspides de la valvule cardiaque du patient pour fixer l'orientation du stent de positionnement (20) par rapport à la valvule cardiaque en direction axiale et en direction horizontale.

4. Dispositif selon la revendication 2 ou 3,
dans lequel le stent de positionnement (20) comprend en outre des éléments de précontrainte (23) pour précontraindre le stent de positionnement (20) en direction radiale dans sa position fixée par l'ancrage (21).

5. Dispositif selon l'une des revendications 2 à 4,
dans lequel le stent de valvule cardiaque (10) est réalisé pour poser la valvule cardiaque du patient contre la paroi du vaisseau à l'aide de la prothèse de valvule cardiaque (11) dans son état en expansion, et ledit au moins un ancrage (21) du stent de positionnement (20) est situé entre la paroi du vaisseau et la valvule cardiaque déployée avec la prothèse de valvule cardiaque (11).

6. Dispositif selon l'une des revendications précédentes,
dans lequel le stent de positionnement (20) comprend au moins un élément d'enclenchement (24) et le stent de valvule cardiaque (10) comprend au moins un élément de maintien (12), pour former une liaison à coopération de forces entre ledit au moins un élément de maintien (12) et ledit au moins un élément d'enclenchement (24) du stent de positionnement (20) dans l'état en expansion du stent de positionnement (20) et dans l'état en expansion complète du stent de valvule cardiaque (10), afin de positionner la prothèse de valvule cardiaque (11) dans la position prédéterminée par le stent de position-nement (20) et de l'y maintenir avec le stent de positionnement (20).

7. Dispositif selon la revendication 6,
dans lequel le stent de valvule cardiaque (10) est réalisé de manière à pouvoir être replié et déployé de faon réversible, et ledit au moins un élément d'enclenchement (24) et ledit au moins un élément de maintien (12) sont ainsi réalisés que, dans l'état replié, la liaison à coopération de forces avec le stent de positionnement (20) est annulée.

8. Dispositif selon l'une des revendications précédentes,
dans lequel des éléments d'explantation (13) sont en outre prévus sur le stent de valvule cardiaque (10), lesdits éléments d'explantation (13) et le stent de valvule cardiaque (10) étant ainsi réalisés que, par une manipulation externe des éléments d'explantation (13), la liaison à coopération de forces entre le stent de valvule cardiaque (10) et le stent de positionnement (20) est annulée et le stent de valvule cardiaque (10) est replié.

9. Dispositif selon la revendication 8,
dans lequel les éléments d'explantation (13) sont ainsi réalisés qu'ils peuvent être saisis à l'aide d'un cathéter (30), les éléments d'explantation (13) étant réalisés de telle façon que par traction des éléments d'explantation (13) en entrant dans le cathéter (30), la liaison à coopération de forces entre le stent de valvule cardiaque (10) et le stent de positionnement (20) peut être annulée et le stent de valvule cardiaque (10) peut être replié.

10. Dispositif selon l'une des revendications 7 à 9,
dans lequel le stent de valvule cardiaque (10) est reçu, dans l'état replié, dans une cartouche (33) susceptible d'être reliée à un cathéter (30), et le stent de valvule cardiaque (10) peut être libéré par un mouvement prédéterminé de la cartouche (33).

11. Dispositif selon la revendication 10,
dans lequel, par un premier mouvement prédéterminé de la cartouche (33), seule la prothèse de valvule cardiaque (11) peut être libérée pour déployer la prothèse de valvule cardiaque (11), et dans lequel, par au moins un second mouvement successif de la cartouche (33) et/ou du cathéter (30), l'élément de maintien (12) peut être libéré.

12. Dispositif selon l'une des revendications précédentes,
dans lequel le stent de positionnement (20) comprend en outre des éléments d'ancrage, en particulier des crochets, pour ancrer le stent de positionnement (20) dans sa position prédéterminée.

13. Dispositif selon l'une des revendications précédentes,
dans lequel le stent de valvule cardiaque (10) comprend en outre des éléments d'ancrage, en particulier des crochets, pour ancrer le stent de valvule cardiaque (10) dans la position prédéterminée avec le stent de positionnement (20).

14. Dispositif selon l'une des revendications précédentes,
dans lequel le stent de positionnement (20) comprend en outre des marqueurs, en particulier des marqueurs pour rayons X, afin de faciliter l'orientation dans l'espace lors de la mise en place du stent de positionnement (20).

15. Dispositif selon l'une des revendications précédentes,
dans lequel le stent de valvule cardiaque (10) est ainsi réalisé qu'il peut occuper son état totalement en expansion, dans lequel aussi bien la prothèse de valvule cardiaque (11) que l'élément de maintien (12) sont libérés, uniquement quand le stent de valvule cardiaque (10) est dans la position prédéterminée par le stent de positionnement (20).

16. Dispositif selon l'une des revendications précédentes,
dans lequel le stent de positionnement (20) et/ou le stent de valvule cardiaque (10) comprennent en outre des organes de guidage (27, 17) pour guider automatiquement le stent de valvule cardiaque (10) dans le stent de positionnement (20) en expansion, jusque dans la position prédéterminée par le stent de positionnement (20).

17. Dispositif selon la revendication 16,
dans lequel les organes de guidage (27, 17) sont réalisés sous la forme d'éléments qui vont en se rétrécissant vers l'extrémité distale du stent de positionnement (20) et/ou du stent de valvule cardiaque (10) pour un ajustement automatique du stent de valvule cardiaque (10) dans le stent de positionnement (20) et ainsi dans la position prédéterminée par le stent de positionnement (20).
